# EUROPEAN PATENT APPLICATION

(11) **EP 2 772 698 A1**
(43) Date of publication of application: **03.09.2014**
(21) Application number: 11874496.0
(22) Date of filing: 25.10.2011
(51) Int. Cl.: F24F 7/00

(54) **FUNCTIONAL AIR CONDITIONING DEVICE AND FUNCTIONAL AIR CONDITIONING METHOD**

(71) Applicant: Panacea Disinfectant Co. Limited, Kowloon, Hong Kong (HK)
(72) Inventor: HATA, Tadayo, Osaka-shi Osaka 545-0053 (JP); HATA, Masuko, Osaka-shi Osaka 545-0053 (JP); HATA, Tomoyo, Osaka-shi Osaka 545-0053 (JP); TOSHIMORI, Yukiko, Osaka-shi Osaka 543-0074 (JP); MARUOKA, Toshiyuki, Toyonaka-shi Osaka 560-0033 (JP)
(74) Representative: HOFFMANN EITLE
(86) International application number: PCT/JP2011/074545
(87) International publication number: WO 2013/061406

(57) **Abstract**

[ABSTRACT] To provide an air-conditioning apparatus and an air-conditioning method with which air can be highly sterilized and cleaned not only in houses, but also in various facilities such as offices and factories, and, safe, pleasant, and healthy living space can be obtained, in a functional air-conditioning apparatus utilizing ground heat provided with a flow piping P which sucks air A in a room R or outside S from a suction opening Pa, sends the air A into ground G to make the air A heat-exchanged with the ground G, and blows the heat-exchanged air A into the room R through a blow opening Pb; the flow piping P is provided with a reciprocating pipe 10 for heat exchange having a going portion 11 to send the air A into the ground G and a returning portion 12 to return the air A heat-exchanged with ground heat under the ground, and a sterilizer 2, in which disinfectant liquid W is reserved, is disposed between the blow opening Pb and a ground exhaust opening 10b of the reciprocating pipe 10 for heat exchange.

## Description

### FIELD OF THE INVENTION

This invention relates to a functional air conditioning apparatus and a functional air conditioning method utilizing ground heat.

### BACKGROUND ART

Conventionally, an air conditioning apparatus and an air conditioning method, utilizing ground heat, sucking air in a room or outside and send the air under the ground, and sending the air heat-exchanged with the ground heat into the room, exist (refer to patent document 1, for example).

### PRIOR ART DOCUMENT

### PATENT DOCUMENT

Patent document 1 : Japanese Patent Provisional Publication NO. 2003-35455.

### OUTLINE OF THE INVENTION

### PROBLEMS TO BE SOLVED BY THE INVENTION

However, the conventional air conditioning apparatus and the air conditioning method utilizing ground heat can't sterilize and clean the air. Although an air cleaner using filter and activated carbon may be set in the room to clean the air, the effect is limited in a large space.

Therefore, it is an object of the present invention to provide an air-conditioning apparatus and an air-conditioning method with which air can be highly sterilized and cleaned not only in houses, but also in various facilities such as offices and factories, and, safe, pleasant, and healthy living space can be obtained.

### MEANS FOR SOLVING THE PROBLEMS

To achieve the object above, a functional air-conditioning apparatus of the present invention is a functional air-conditioning apparatus utilizing ground heat provided with a flow piping which sucks air in a room or outside from a suction opening, sends the air into ground to make the air heat-exchanged with the ground, and blows the heat-exchanged air into the room through a blow opening wherein; the flow piping is provided with a reciprocating pipe for heat exchange having a going portion to send the air into the ground and a returning portion to return the air heat-exchanged with ground heat under the ground, and a sterilizer, in which disinfectant liquid is reserved, is disposed between the blow opening and a ground exhaust opening of the reciprocating pipe for heat exchange.

And, in the functional air-conditioning apparatus, the sterilizer has a heat insulating casing for sterilization, a liquid reserve portion in which the disinfectant liquid is reserved, and a filter of which one end side is dipped to the disinfectant liquid in the liquid reserve portion to suck the disinfectant liquid by capillary phenomenon; and the sterilizer has an intake guide portion to induce the flowing air to the liquid reserve portion side between an inflow opening of the casing for sterilization and the filter.

And, the filter is composed of non-organic fiber of which thickness is set to be 0.01mm to 0.05mm, and the thickness of the filter is set to be 0.05mm to 2.0mm.

And, the disinfectant liquid has metal ion having antibacterial function, L-cysteine, and L-ascorbic acid as main components, and includes surface active agent except non-ion system.

And, the sterilizer is provided with a temperature control means to control the temperature of the disinfectant liquid, and temperature of the air blown out of the sterilizer is controlled by the temperature control of the disinfectant liquid.

And, a physiology activator, having a casing for storage with heat insulation having a connection opening in which the air exhausted from the sterilizer, a storing portion in which physiology activating matter effective for resolving stress is stored, and an ultrasonic wave generating means to volatilize the physiology activating matter to be mixed with the air, is disposed between the blow opening and the sterilizer.

And, the air-conditioning method of the present invention is a functional air-conditioning method utilizing ground heat to send air in a room or outside into ground to make the air heat-exchanged with the ground, and send the heat-exchanged air into the room wherein the air is made contact with stored disinfectant liquid to be sterilized and cleaned before being sent to the room.

And, the air is induced to the disinfectant liquid for contact on the ground side of a flow piping in which the air flows, and the air is made pass through a filter sucking the stored disinfectant liquid by capillary phenomenon to be sterilized and cleaned.

And, in a functional air-conditioning method utilizing ground heat to send air in a room or outside into ground to make the air heat-exchanged with the ground, and send the heat-exchanged air into the room; an inner peripheral face of the flow piping in which the air flows is made wet with the disinfectant liquid, and the air is made contact with the disinfectant liquid and flow in the flow piping to be sterilized and cleaned.

And, a sterilizer, in which the disinfectant liquid is stored, is disposed on the way of the flow piping to exhaust the air out of a blow opening to the room.

### EFFECTS OF THE INVENTION

According to the present invention, clean, clear, and high quality germ and odor free air, in which dust, pollen, carcasses of mite, hair of companion animals, etc. hardly exist, can be sent to the room, and safe, pleasant, and healthy living space can be obtained. High level air cleanliness and healthy life can be obtained not only in houses, but also in offices and factories, and various large facilities.

And, in comparison with conventional air-conditioning apparatus and method utilizing ground heat, further excellent functions are shown to contribute to popularization of air-conditioning apparatus utilizing ground heat. Although technology to take the ground heat, cool in summer and warm in winter in comparison with open air, effectively with heat-exchange pipes and heat pumps is basically established and running cost of factories can be reduced for 30% to 40% in energy efficiency, popularization of the apparatus is hardly progressing because of the cost problem for installment of the apparatus with drilling work of the ground. To popularize the air-conditioning apparatus and method utilizing ground heat under the current conditions, it is necessary not only energy saving effect, but also supplying unconventionally good and high quality air. Active inducement of recyclable energy is socially discussed along with environmental problem, sense of security and safety comes first, and people are eagerly looking for disease prevention, healthy long life, and return to nature. In Japan, becoming super aged society, these inclinations are becoming more and more remarkable, the apparatus will be accepted with agreement of many people as to compensate the high initial cost.

And, now is the age in which "sense of security and safety" is considered more important than any other things throughout the society. Some people say that the vast land around Fukushima atomic power plant may be uninhabitable for hundred years for radioactive leakage. Now we are entering to the age in which environmental problem and ecology issues are indispensable. Ground resource is limited and oil depletion is not so far. However, natural energy (solar heat, solar ray, wave, water power, etc.) is sustainable as long as the earth exists.

One of the energy is ground heat. The earth is a gigantic heat-accumulating layer, it is suitable to eco-conscious age to take the ground heat source (warm in winter and cool in summer) to utilize greatly for air-conditioning apparatus and method, and it is a social responsibility to promote the popularization of the apparatus and method. The present invention is not only eco-conscious, but also able to easily obtain "sense of security, safety, and health".

### BRIEF DESCRIPTION OF THE DRAWINGS

[Figure 1] A simplified constructional view showing an embodiment of a functional air-conditioning apparatus and functional air-conditioning method of the present invention.
[Figure 2] A cross-sectional side view of a principal portion showing an example of a sterilizer and a physiology activator.
[Figure 3] A simplified constructional view showing another embodiment of the functional air-conditioning method of the present invention.

### EMBODIMENTS OF THE INVENTION

The present invention will now be described according to the embodiments shown in the drawings.

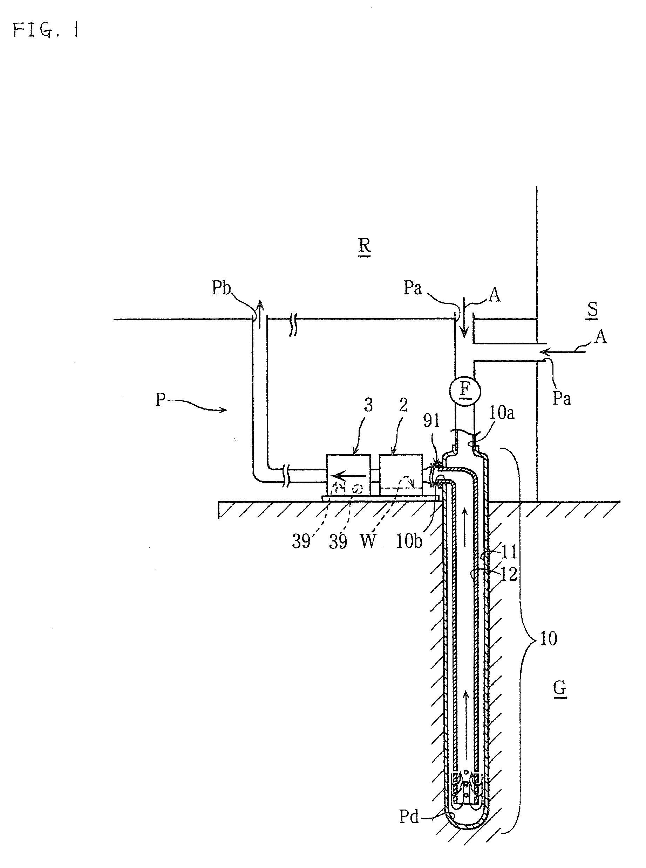

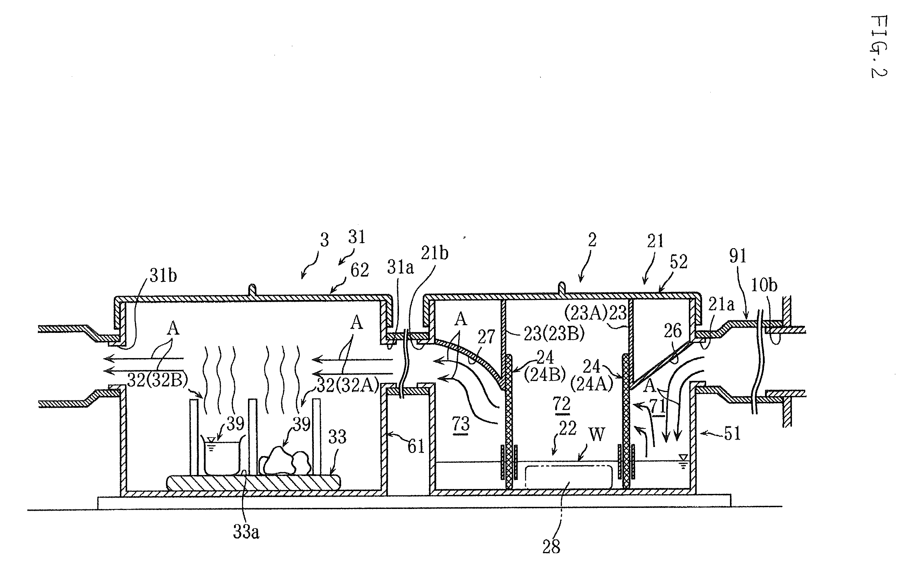

The functional air-conditioning apparatus relating to the present invention is, in an embodiment shown in Figure 1 and Figure 2, a functional air-conditioning apparatus utilizing ground heat provided with a flow piping P having a suction opening Pa to suck air A in a room R of a building or outside S, a reciprocating pipe 10 for heat exchange to send the air A into ground G for heat exchange with ground heat and to send the air A again over the ground, and a blow opening Pb to exhaust the air A heat-exchanged with ground heat under the ground G into the room R.

The heat-exchange reciprocating pipe 10 (also called the reciprocating pipe 10 below), having a ground air supply opening 10a connected to the suction opening Pa and to which the air A flows through a fan F, a going portion 11 to send the air over the ground into the ground, a returning portion 12 to return the air A heat-exchanged under the ground, and a ground exhaust opening 10b to send the heat-exchanged air A to the blow opening Pb, is constructed as a double pipe and embedded along a vertical axis.

And, a sterilizer 2, in which disinfectant liquid (multi-function sterilization and disinfectant liquid) W is stored, is disposed between the blow opening Pb and the ground exhaust opening 10b of the reciprocating pipe 10.

In Figure 2, the sterilizer 2 has a casing 21 for sterilization having heat insulation.

The casing 21 for sterilization is provided with an upper opening box member 51 for sterilization having an inflow opening 21a to which the air A enters and an outflow opening 21b to let the air A flow out, and a lid member 52 for sterilization to lid the box member 51 for sterilization.

And, the sterilizer 2 is provided with a liquid reserve portion 22 to reserve the disinfectant liquid W on the bottom portion of the box member 51 for sterilization, two filters 24 of which end side is dipped to the disinfectant liquid W in the liquid reserve portion 22 for sucking the disinfectant liquid W by capillary phenomenon, and two parting portions 23 of vertical wall to divide the casing 21 for sterilization into 3 chambers with the filters 24 disposed as vertical walls.

Further, the sterilizer 2 has an intake guide portion 26 to guide the inflow air A (downward) to the liquid reserve portion 22 between the inflow opening 21a and the filter 24 (also called the intake filter 24A below) on the inflow opening 21a side.

The intake guide portion 26 is formed inclined downward to the front in side view and continued to the intake parting portion 23 (23A) as to the inflow air A easily contact with the surface of the disinfectant liquid W.

And, it is preferable to set the diameter of the inflow opening 21a smaller than average inner diameter of a connecting pipe 91 to connect the ground exhaust opening 10b of the reciprocating pipe 10 and the inflow opening 21a as to increase the flowing speed of the inflow air A.

And, an exit guide portion 27 to guide the air A on the disinfectant liquid W (lower portion) side to the outflow opening 21b is formed between the outflow opening 21b and the filter 24 (also called the exit filter 24B below) on the outflow opening 21b side. The exit guide portion 27 is continuous from the exit parting portion 23 (23B) and formed into an arc of quarter circle (concave curved face). The parting portions 23 (23A, 23B), the intake guide portion 26, and the exit guide portion 27 are formed as a plate.

And, the sterilizer 2 has a first space (chamber) 71 between the inflow opening 21a and the intake filter 24A, a second space (chamber) 72 between the intake filter 24A and the exit filter 24B, and a third space (chamber) 73 between the exit filter 24B and the outflow opening 21b. And, the disinfectant liquid W and the filters 24 can be changed and supplied by removal of the lid member 52 for sterilization.

And, the filter 24 is disposed as to be freely inserted and removed (detachably attached) in vertical direction to and from the box member 51 for sterilization.

The material of the filter 24 is non-organic fiber which has advantages that thickness, length, configuration, and density can be easily set in comparison with natural cotton and cellulose, and deterioration is low.

Concretely, the material is glass wool, rock wool, metal wool, etc. The thickness of the fiber is set to be 0.01mm to 0.05mm, and the thickness of the filter 24 is set to be 0.05mm to 2.0mm. And, in case of glass wool, density is 20 to 30g/1000cm³, in case of rock wool, density is 15 to 20g/1000cm³, and in case of metal wool, density is 40 to 60g/1000cm³.

And, the sterilizer 2 is provided with a temperature control means 28 to control the temperature of the air A exhausted from the outflow opening 21b by controlling the temperature of the disinfectant liquid W within the liquid reserve portion 22.

Concretely, the temperature control means 28 is a cooling body such as a cold insulator stored in a sealed case when the temperature of the exhausted air A is made lower than that of the inflow air A, and a heat emitting body such as a heat insulator, a hot-water bag, etc. stored in a sealed case when the temperature of the exhausted air A is made higher than that of the inflow air A.

Especially, in case that the temperature of the exhausted air A is made higher than that of the inflow air A, the temperature of the disinfectant liquid W is set to be higher than that of the inflow air A for more than 15°C, to increase the temperature of the exhausted air A for 1.5 to 2.5°C, more preferably, for 3°C. It is desirable to set the temperature of the disinfectant liquid W equal to or less than 45 °C , or temperature difference between the disinfectant liquid W and the air A is equal to or less than 40°C.

And, in case that the temperature of the exhausted air A is made lower than that of the inflow air A, the temperature of the disinfectant liquid W is set to be lower than that of the inflow air A for more than 5 °C, to decrease the temperature of the exhausted air A for 1.5 to 2.5°C, more preferably, for 3°C. It is desirable to set the temperature of the disinfectant liquid W equal to or more than 5 °C, or temperature difference between the disinfectant liquid W and the inflow air A is equal to or less than 15°C.

Further, as shown in Figure 1, in the flow piping P, a physiology activator 3 to mix volatilized physiology activating matter 39 with the air A is disposed between the blow opening Pb and the sterilizer 2.

The physiology activator 3 contributes to remedy for chronic unpleasant symptoms caused by stress, and resuscitation and improvement of natural healing power by resolving the stress.

In Figure 2, the physiology activator 3 has a casing 31 for storage having heat insulation.

The casing 31 for storage is provided with a connection opening 31a to which the air A exhausted from the sterilizer 2 flows in, an upper opening box member 61 for storage having an exit 31b to let the inflow air A flow out, and a lid member 62 for storage to lid the box member 61 for storage. The physiology activating matter 39 is easily changed and supplied by removal of the lid body 62.

Then, the physiology activator 3 is provided with a storing portion 32 in which the physiology activating matter 39, effective for resolving stress, is stored within the box member 61 for storage, and an ultrasonic wave generating means 33 disposed on the bottom portion of the box member 61 for storage to volatilize and mix the components (extracts, odors, etc.) of the physiology activating matter (healing matter) 39 with the air A flowing into the upper part of the casing 31 for storage.

The ultrasonic wave generating means 33 may be constructed as to change (set) frequencies of the ultrasonic wave to induce evasive action of unpleasant pestilence such as rats, cockroaches, wild cats, pigeons, etc. Upper opening plural storing portions 32 (32A, 32B), divided by plural parting members, are formed on a horizontal placement face 33a of the ultrasonic wave generating means 33. To make the inflow air A warmer or cooler further, increasing means of coolness and warmness of the air composed of things warmer or cooler than the air A (cooling body, heat emitting body, etc.) may be stored in the storing portion 32.

The physiology activating matter 39, effective for resolving (remedy) the stress, includes matters to resolve the stress by placebo effect with believing.

Concretely, the physiology activating matter 39 is essential oil (extract) used for aromatherapy (odor therapy). Aromatherapy is a kind of alternative medicines utilizing essences extracted from odoriferous plants to refresh mind and body, and raise natural healing power to make people healthy in which the essences used corresponding to the types of stress as to generate relaxation (stress resolving) effect.

The sense of smell is transmitted to the limbic system through skin and nasal cavity, connected with old memories and instinctive acts, and transmitted further to hypothalamus to generate physiological reaction. Hypothalamus is an important organ even called "the brain in the brain" to control autonomous nerves, endocrine systems, and immunological systems as to maintain the homeostasis of the body. The sense of smell is directly connected to bodily physiological reaction without intellectual cerebral cortex. Examples of the essential oil (extract) effective to stress-generated chronic diseases and their physiological reactions except resolving stress are shown in Table 1 below.

And, volatile matters (forest odor, phytoncide) emitted by trees, chips of cedar and hinoki cypress, and pine leaves are similar, and, simple smell such as smell of hay, rice straw, smell of memories in infant age, nostalgic smell, and favorite odor of chocolate, etc. compose a kind of aromatherapy (extract).

**[Table 1]**

| | Chronic Disease | Physiological Functions |
|---|---|---|
| Ilang-ilang | High blood pressure | Irritation, insomnia, sedation |
| Camomile | urticaria | Anxiety, tonus, sedation |
| Rose | Mensural disorder | Insomnia, skin trouble |
| Peppermint | Paranasal sinusitis | Neurogastritis, constipation |
| Marjoram | constipation | Headache, muscle pain, eye strain |
| Neroli | Chronic diarrhea | Remedy to depression, hypnosis, sedation |
| Lavender | alopecia | Cold, dysmenorrhea, arthralgia |
| Benzoin | asthma | Sleep, pain killing, itch |
| Camphor | Low blood pressure | Blood circular promotion, Sedation, antiphlogistic, cardiotonic |
| Lemon Balm | Meniere's disease | Sedation, eczema, high blood pressure |
| Phytoncide | | Bacteria resistance, Bactericide, improvement of hepatitis function, autonomous stability, tumor, deodorance, Insecticide(tick) |

And, although actual effect (scientific effect) is not established and having unclear points, the physiology activating matter 39 may be so-called medicine stone.

Examples of medicine stones (minerals), matters, and their micro powder considered effective to resolving stress and their physiological reactions except resolving stress are shown in Table 2 below.

**[Table 2]**

| | Physiological Functions |
|---|---|
| Soraseki | Antibacterial function, antiphlogistic function, far infrared effect, improvement of soils |
| Maifan stone | Dermatitis, sedation, bacteria removal, deodorant |
| Tourmaline | Blood circular promotion, recovery from fatigue, sleep |
| Radium(radon)crude | Diabetes, high blood pressure, asthma |
| Iouseki stone | sedation |
| Binchotan charcoal | Far infrared, humidity control, deodorant, absorbing harmful matters |

And, the physiology activating matter 39 may be musk, ambergris, etc. as animal matters.

And, the physiology activating matter 39 may be anything that people in the room R believe good to them such as special salt, special water, etc. even without scientific basis. The matter induces placebo effect. It is a widely known fact that this is effective. And, in the present invention, volatilization means dispersion of components which the physiology activating matter 39 includes, such as dispersion with gasification, dispersion of micro particles, dispersion of odorous components, etc.

Next, the disinfectant liquid W, of which main components are amino acid as basic matter of life and life action, vitamins, and minerals, having very high safety, wide antibacterial specter from general bacteria, anti acid bacteria, germs, mycobacteria, to viruses, can be used multi-purpose as to be applied to hands and fingers, mucous membrane, wound, instruments, facilities, excrement, environment, and to fresh foods and agricultural products further.

The disinfectant liquid W has concretely one or more than two kinds of metal ions having antibacterial function, L-cysteine, and L-ascorbic acid as main components, and one or more than two kinds of surface active agents except non-ion system are added.

The metal ions having antibacterial function are triatomic iron ion (Fe3+), diatomic iron ion (Fe2+), zinc ion (Zn2+), copper ion (Cu2+), cobalt ion (Co2+), nickel ion (Ni2+), and silver ion (Ag+).

And, density of the metal ion having antibacterial function is 50 to 200ppm for triatomic iron ion, 110 to 400ppm for diatomic iron ion, 7.5 to 125ppm for zinc ion, 15 to 60ppm for copper ion, 180 to 300ppm for cobalt ion, 85 to 175ppm for nickel ion, and 1 to 3ppm for silver ion.

And, density of L-cysteine is 100 to 1000ppm, and density of L-ascorbic acid is 100 to 500ppm.

And, the surface active agents except non-ion system are more than one kind selected from a group composed of alkylbenzen sulfate, normal chain alkylbenzen sulfate, polyoxyethylene alkylether sulfate, higher alcohol sulfate, lauryl sodium sulfate, lauryl sarcosine sodium, stearil dimethylbenzen ammonium chloride, benzalkonium chloride, benzenthonium chloride, alkyldiaminoethylglycine hydrochloride, and alkylpolyaminoethylglycine hydrochloride.

And, density of the surface active agents except non-ion system is 20 to 100ppm.

And, surface active agents further include at least one kind selected from a group composed of sorbic acid, sorbic acid chloride, benzoic acid, benzoate, and paraoxy benzoate.

And, small amount of various vegetable essential oils and antibacterial components such as of hinoki cypress, mint, etc. or mineral antibacterial components may be added to the above-described disinfectant liquid W to enhance the antibacterial power.

Next, production examples of the above-described disinfectant liquid W are described.

In production example 1, 0.96g of ferric chloride hexahydrate is dissolved into 200ml of purified water as first solution. 1g of L-cysteine, 0.1g of L-ascorbic acid, 0.05g of sorbic acid potassium, and 0.1g of lauryl sodium sulfate are dissolved into 800ml of purified water as second solution. Then, the first solution and the second solution are mixed, 1ml of 1 normal hydrochloric acid is added to produce the disinfectant liquid W composed of the components below.

The components are 200ppm of Fe3+ ion, 1000ppm of L-cysteine, 100ppm of L-ascorbic acid, 50ppm of sorbic acid potassium, and 100ppm of lauryl sodium sulfate. And, PH is 3.0.

Production examples 2 to 4 are shown below in Table 3.

**[Table 3]**

| Ex | Mineral component | | L-cysteine density | L-ascorbic Acid density | Kind and density of Surface active agent | Kind and density of food presavative | PH |
|---|---|---|---|---|---|---|---|
| | Compound | Ion density | | | | | |
| 2 | CoCl₂ · 6H₂O 0.45g | Co²⁺ 100ppm | 500ppm | 200ppm | Benzalkonium Chloride 100ppm | Sodium Benzoate 50ppm | 4.0 |
| | NiSO₄ · 7H₂O 0. 48g | Ni²⁺ 100ppm | | | | | |
| 3 | ZnSO₄ · 7H₂O 0.44g | Zn²⁺ 100ppm | 500ppm | 100ppm | Lauryl sodium sulfate 50ppm | | 3.5 |
| | AgNO₃ 0.0026g | Ag+ 2ppm | | | polyoxi ethylene alkylether sulfate 50ppm | | |
| | NiSO₄ · 7H₂O 0.48g | Ni²⁺ 100ppm | | | | | |
| 4 | FeCl₃ · 6H₂O 0.75g | Fe³⁺ 150ppm | 1000ppm | 100ppm | Lauryl sodium sulfate 100ppm | Sorbic acid Potassium 50ppm | 3.0 |
| | ZnSO₄ · 7H₂O 0.1g | Zn²⁺ 23ppm | | | | | |
| | NiSO₄ · 7H₂O 0.24g | Ni²⁺ 50ppm | | | | | |

Next, controlled suspension liquid of various kinds of bacteria (approximately 1 × 10⁹ cells/physiological salt water) of 2% by weight is dropped into the disinfectant liquid of production example 3, inoculated by serial fishing with platinum loops to culture medium for breeding, and cultured under optimum circumstances, and sterilization effect is observed by breeding of bacteria. General bacteria (pneumococcus, diphtheria, B-coli, salmonella, etc.) are destroyed in 10 to 15 seconds, anti-acid bacteria (human-type tuberculosis, etc.) are destroyed in 1 minute. And, fungus (candida, etc.) of filamentous type is destroyed around for 15 seconds, and yeast type is destroyed around for 30 seconds. Spores (herbicide bacteria, etc.) are destroyed (deactivated) in 1 to 120 minutes corresponding to their forming stages.

In case of viruses, avian influenza H5N3 type having envelope decreased titer (activeness) to 1/100,000 to 1/1,000,000 by contact of 5 minutes. In case of norovirus not having envelope, when infected oyster and excrement of infectant are tested by dipping into disinfectant liquid of amount 10 times larger than them, the virus is destroyed (deactivated) after 30 minutes.

Next, working of the functional air-conditioning apparatus of the present invention and the functional air-conditioning method of the present invention will be described.

In Figure 1, the air A in the room R or outside S is sent to the going portion (outer cylinder portion) 11 through the ground air supply opening 10a of the reciprocating pipe (double pipe for heat exchange with ground heat) 10. The air A flowing the going portion 11 turns on the bottom portion Pd of the reciprocating pipe 10, flows through the returning portion (inner cylinder portion) 12, and flows into the connecting pipe 91 through the ground exhaust opening 10b. The air A is heat-exchanged during the flowing through the ground G to be cooled in summer, and warmed in winter.

In Figure 2, the heat-exchanged air A flows through the connecting pipe 91 and flows into the sterilizer 2. The inflow air A is guided to the surface of the disinfectant liquid W by the intake guide portion 26 in the first space 71, and dust, pollen, fungi, hair of companion animals, etc. are absorbed to be removed or sterilized by direct contact with the surface of the liquid. Further, the air A passes through the intake filter 24A. When the air A is passing, foreign matter is sterilized, cleaned, and absorbed by the disinfectant liquid W sucked up by capillary phenomenon, and absorbed by the fiber of the intake filter 24A.

The air A which passed the intake filter 24A passes the exit filter 24B through the second space 72, remaining foreign matter, which is not removed by the intake filter 24A, is absorbed, removed, and sterilized. The sterilized (cleaned) air A is guided smoothly to the outflow opening 21b by the exit guide portion 27, and sent to the physiology activator 3.

And, in the sterilizer 2, the air A is cooled in summer and warmed in winter by heat exchange with the disinfectant liquid W temperature-controlled by the temperature control means 28.

The cleaned air A exhausted from the sterilizer 2 is mixed with the physiology activating matter 39 volatilized by the ultrasonic wave generating means 33 in the physiology activator 3. The air A sterilized, cleaned, and including the components (stress-dissolving components) of the physiology activating matter 39, is exhausted to the room R to provide people with sense of security, safety, pleasant, and healthy life.

That is to say, the functional air-conditioning method of the present invention is a functional air-conditioning method, utilizing ground heat to send the air A in the room R or outside S into the ground G to make the air A heat-exchanged with the ground G, and send the heat-exchanged air A into the room R, in which the air A is guided to the liquid surface side to be made contact with the stored disinfectant liquid W on the ground side and the blow opening Pb side of the flow piping P in which the air A flows before being sent to the room R, the air A is sterilized and cleaned by serial passing through the two filters 24 sucking up the disinfectant liquid W by capillary phenomenon, the temperature of the air A is controlled by heat exchange with the disinfectant liquid W temperature-controlled by the temperature control means 28, and then, the sterilized and temperature-controlled air A is mixed with the (components of) physiology activating matter 39 volatilized by the ultrasonic wave generating means 33 and sent to the room R.

Next, function (functional test) of the sterilizer will be described.

The sterilizer 2 is set in the room R, bacteria (lactobacillus of which number is easily measured, harmless B-coli, and spores of blue mold), pollen (of cedar and hinoki cypress), dust, carcasses of mites, components of bad smell (excrement, garbage, ammonia, hydrogen sulfate, trimethylamine smelling like rotten fish) are sprayed and stirred by fans on the four corners of the room R to float with drying, and, degree of cleanliness of the air A in the room R and in the sterilizer 2 (the second space 72 and the third space 73) and differences of smell and temperature of the air A are measured (test example 1). The test is conducted with suction from the outflow opening 21b of the sterilizer 2 with negative pressure (suction power of 60W). The room temperature is set to be 15°C.

In case of bacteria, 300ml of the air A in each place (in the room R, the second space 72, and the third space 73) is collected by a sterilized syringe of which capacity is 500ml, 100ml of sterilized physiological salt water is sucked by the same syringe, stirred well to make the physiological salt water absorb the bacteria in the air A, and number of bacteria is measured with selected culture medium according to ordinary method. For example LBS culture medium for lactobacillus, EC culture medium for B-coli, and sabouraud agar for fungi.

In case of pollens and carcasses of mites, Vaseline is painted uniformly on a slide glass (26mm × 76mm) with 1mm mesh, and 100ml of the air A collected in a glove box is uniformly blown to the slide glass. The pollen is dyed with gentian violet glycerol jelly, observed under microscope with magnification of 100 to 200 times, and the carcasses of mites are observed under magnification of 5 to 10 times.

In case of dust, collected air A is put into a clean glass box, and light is shed with a background of black paper to observe dust in Tyndall phenomenon by naked eye.

In case of components of bad smell, air A is collected by a syringe of 500ml and put in a smelling (odor) bag, and average value of organoleptic test of five persons is obtained.

And, degree of the dust is valued with 6 stages as shown below. 5 + marks mean dusty as the background is almost unseeable. 4 + marks mean the background is seeable but dusty. 3 + mark means normal (as in public spaces). 2 + marks mean little (as in a normal room). One + mark means very little and clean and beautiful to eyes. And one -mark means perfectly clean.

And, degree of bad smell is valued with 6 stages as shown below. 0 means no smell. 1 means slightly sensed smell. 2 means easily (little) sensed smell. 3 means clearly sensed smell. 4 means strong smell. And 5 means unbearably strong sensed smell.

The measuring results are shown below in Table 4.

**[Table 4]**

| Test item | In the room | In the sterilizer | |
|---|---|---|---|
| | | Air passed the intake filter (in the second space) | Air passed the exit filter (in the third space) |
| ①Bacteria, Fungi Number of | | | |
| B-coli | 150 | 18 | 1 |
| Lactobacillus | 120 | 15 | 0 |
| Fungi(spores) | 75 | 2 | 0 |
| ②Number of Pollen/cm² (cedar, hinoki) | 225 | 0 | 0 |
| ③Carcasses of mites | 8 | 0 | 0 |
| ④degree of dust | (++++) | (- ∼ +) | (-) |
| ⑤Smell of | | | |
| Excrement | 3 | 1 | 0 |
| Garbage | 3 | 0 | 0 |
| Ammonia | 3 | 1 | 0 |
| Hydrogen sulfate | 3 | 1 | 0 ∼ 1 |
| Trimethylamine | 4 | 3 | 1 |

As clearly shown in Table 4, bacteria floating in the air A, even passing through the intake filter 24A, are mostly caught and sterilized by the exit filter 24B (reduced for more than 99.6%). And, 100% of pollens and mites are caught, and smell is also removed. And, although not shown in Table 4, hair of companion animals is also removed.

And, anti-acid bacteria such as tuberculosis, spores of bacteria, influenza B type, avian influenza H1N1 type, norovirus, etc. were also tested, and it was confirmed that almost 100% of them, similar to the above-mentioned bacteria and fungi, are absorbed by the filter 24 and sterilized or deactivated.

And, the smell, depending on the character of the smell, of garbage and excrement, which are generated in daily life, is mostly deodorized. Although smell rather difficult to be solved into water such as smell of hydrogen sulfate and trimethylamine slightly remain, the smell is removed when the remaining smelly air is returned to the sterilizer 2 for 1 to several times. That is to say, when the air A is circulated, most of the smell is eventually absorbed, and clear air A without smell can be obtained.

Next, the room temperature is set to be predetermined temperature, temperature of each place (in the room R, the second space 72, and the third space 73) is measured with changing the temperature of the disinfectant liquid W by the temperature control means 28. The measured results are shown in Table 5 below.

**[Table 5]**

| Room temperature | Temperature of Disinfectant liquid | In the sterilizer | |
|---|---|---|---|
| | | First space | Second space |
| 10.0°C | 10.0°C | 9.5°C | 9. 2°C |
| | 15.0°C | 9.8°C | 9.8°C |
| | 20.0°C | 11.0°C | 10.8°C |
| | 25.0°C | 11.5°C | 11.5°C |
| | 30.0°C | 12.0°C | 12.2°C |
| | 40.0°C | 12.5°C | 13.0°C |
| 15.0°C | 10.0°C | 13.8°C | 13.5°C |
| | 15.0°C | 14.2°C | 13.8°C |
| | 20.0°C | 15.0°C | 15.0°C |
| | 25.0°C | 15.8°C | 16.0°C |
| | 30.0°C | 16.5°C | 17.0°C |
| | 40.0°C | 17.5°C | 18.0°C |
| 20.0°C | 10.0°C | 18.0°C | 17.5°C |
| | 15.0°C | 18.8°C | 18.0°C |
| | 20.0°C | 19.0°C | 18.6°C |
| | 25.0°C | 20.0°C | 20.0°C |
| | 30.0°C | 21.0°C | 21.2°C |
| | 40.0°C | 22.0°C | 22.5°C |
| 25.0°C | 10.0°C | 22.5°C | 21.7°C |
| | 15.0°C | 23.6°C | 23.2°C |
| | 20.0°C | 23.8°C | 23.5°C |
| | 25.0°C | 24.0°C | 23.5°C |
| | 30.0°C | 25.0°C | 25.0°C |
| | 40.0°C | 26.5°C | 27.0°C |

As clearly shown in above Table 5, when the temperature of the disinfectant liquid W is set to be higher than that of the inflow air A (room temperature) for more than 15°C, the exhausted air A is certainly warmed for 1.5 to 3.0°C. And, when the temperature of the disinfectant liquid W is set to be lower than that of the inflow air A for more than 5 °C , the exhausted air (the air passed through the exit filter 24B) A is certainly cooled for 1.5 to 3.0°C.

In other words, when the room temperature (the temperature of the inflow air A) is equal to the temperature of the disinfectant liquid W, the air (exhausted air) A passed through the exit filter 24B is cooled for 0.8 to 1.5°C. And, as the temperature of the disinfectant liquid W is made lower than the room temperature, the temperature of the air A passed through the exit filter 24B is made lower.

And, when the temperature of the liquid is higher than the room temperature for 5 °C, the temperature of the air A passed through the exit filter 24B is made approximately equal to the room temperature (with difference smaller than 0.5°C). And, when the temperature of the liquid is higher than the room temperature for more than 5°C, it can be said that as the temperature of the disinfectant liquid W is made higher than the room temperature, the temperature of the air A passed through the exit filter 24B can be made higher.

And, when the distance between the intake filter 24A and the exit filter 24B is changed, the temperature of the air A passed through the exit filter 24B slightly varies.

And, in the room R similar to that of the test example 1, bacteria floating in the air are collected by sucking with a air-floating bacteria sampler (MBS-1000, collection efficiency is more than 99% in JIS test), blown with high speed to a nutrition culture medium of agar to culture, and number of bacteria is measured (test example 2).

In each of the second space 72 and the third space 73, 25L of the air A is collected by the work of the sampler for 15 seconds and blown to the culture medium. The results in case that the number of bacteria in the room R is 100 is shown in Table 6 below.

**[Table 6]**

| Kind of microbe | In the room | In the sterilizer | |
|---|---|---|---|
| | | Second space | Third space |
| General bacteria | 100 | 0 | 0 |
| Spores | 100 | 5 | 0 |
| Fungi | 100 | 2 | 0 |

As clearly shown in above Table 6, spores and fungi passed through the intake filter 24A are completely caught by the exit filter 24B. And, when pollens and carcasses of mites are collected by the same sampler and measured, they are also completely caught by the exit filter 24B, and not detected in the third space 73. And, beautiful air without dust is obtained.

Further, considering a case that the air A in the outside S, the sterilizer is set in the outside S (garden) to be tested (test example 3). Collection of the air A in the third space 73 by the above-mentioned sampler results that similar tendency to that of the case in which the sterilizer 2 is set in the room R, the air A passed through the exit filter 24B is approximately germ free, and clean air is obtained without pollen and mites. And, hair of companion animals can be removed.

As clearly shown by the above-described test results, beautifully clear air approximately germ and odor free can be obtained also in case that the sterilizer 2 is disposed on the flow piping P. And, it is also effective as an energy saving measure because cooler air A is obtained in summer and warmer air A is obtained in winter. Atmosphere as in a pleasant plateau is obtained by natural coolness not only in general households, but also institutions for elderly people and physically weak people. And, in winter period (season), adjustment of clothes and appropriate physical activity can cope with the winter season without heating machines. And, not restricted to the ground heat, the sterilizer 2 may be disposed on air-conditioning ducts of an air-conditioning apparatus using boilers and heat pumps to obtain the above-described clean air A.

And, as shown in Figure 1, the case, in which the sterilizer 2 is disposed on the flow piping P which sucks the air A in the room R or outside S and send the air A heat-exchanged with the ground heat into the room R, is tested. The air A in the outside S can be heat-exchanged with the ground heat of depth of 5m by the reciprocating pipe 10. And, although not shown in Figures, water for absorbing dust, etc. is reserved in the bottom portion Pd of the reciprocating pipe 10.

Number of bacteria, fungi, pollens, degree of dust, carcasses of mites, garbage smell, and temperature are measured (metered) in the room R similar to that of the test example 1. The temperature of the disinfectant liquid W is set to be 10°C (lower than the air A flowing into the sterilizer 2 for 8°C). The results are shown in Table 7 below. The method for collecting and testing the air A is similar to that of the test example 1.

**[Table 7]**

| | Outside air | Just before Entering the sterilizer | After the sterilizer works | |
|---|---|---|---|---|
| | | | Second space | Third space |
| Number of bacteria | 135 | 42 | 0 | 0 |
| Number of fungi | 56 | 18 | 0 | 0 |
| pollens | 215 | 55 | 0 | 0 |
| Carcasses of mites | 5 | 1 | 0 | 0 |
| Dust degree | (+++) | (+) | (-) | (-) |
| Smell (of garbage) | 3 | 2 | 1 | 0 |
| temperature | 25°C | 18°C | 16.5°C | 15.8°C |

As clearly shown in above Table 7, the air A is remarkably cleaned, and beautiful air A without smell is obtained. Although the air A flowing into the sterilizer 2, in comparison with the outside air, has number of bacteria reduced for 69%, number of fungi reduced for 68%, pollens reduced for 75%, carcasses of mites reduced for 80%, and dust and smell reduced to the half, and temperature of the air A is decreased by heat exchange with ground heat from 25 °C to 18°C, germ and odor free and clearer air A is obtained by setting of the sterilizer 2, and temperature control effect is also improved. And, hair of companion animals can be also removed.

And, as shown in Figure 1, the case, in which the physiology activator 3 is disposed, is described.

The physiology activating matters 39, corresponding to preferences of 20 examinees annoyed by stress-generated chronic unpleasant symptoms, are stored in the physiology activator 3 connected to each of the rooms R and volatilized, and 4 weeks of stay of the examinees, although the results vary among the individuals, revealed that the symptoms of the chronic diseases are softened in all examinees. Among the examinees, complete disappearance of the symptom is also reported. The faces of the examinees become mild and bright in external appearances, it is considered that high effect is shown with the almost germ free, highly cleaned air A.

The current society is characterized by the stress prevailing in the society. As the reverse side of this, "sense of security, safety, and preference of health" are considered important, it can be said that modern people are seeking for exodus from the stressful society. The great stress beyond solution of individuals such as strictly competitive society, excessive information, sense of loneliness in home and workplace, difficulty of human relations, etc. makes bodily disorder, and the weakest point of people is revealed as disease. This expands to various fields of the body, for example, high blood pressure, low blood pressure, varicosity, urticaria, headache, obesity, mensural disorder and excessiveness, paranasal sinusitis, meteorism, chronic diarrhea, constipation, insomnia, alopecia, asthma, skin trouble, etc. This shows the reason of the saying that stress is cause of all diseases.

There is no panacea for chronic diseases caused by stress, and the most important points to softening symptoms and cures are liberation from stress, mental stability, and mental healing. Because, mental stability can promote natural healing power and immunity.

Next, another embodiment of the functional air-conditioning method of the present invention is described.

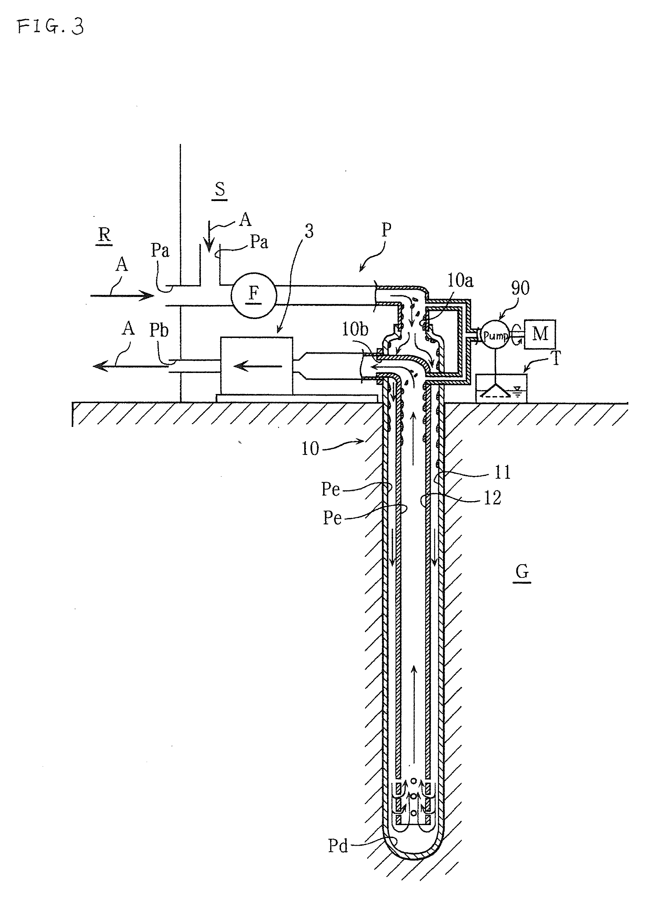

As shown in Figure 3, in a functional air-conditioning method utilizing ground heat provided with the flow piping P to send the air A in the room R or outside S into the ground G to make the air A heat-exchanged with the ground G and send the heat-exchanged air A into the room R, the inner peripheral face Pe of the flow piping P (the reciprocating pipe 10) in which the air A flows is made wet with the disinfectant liquid W, the air A is made contact with the disinfectant liquid W and sent in the flow piping P to be sterilized and cleaned. And, a small amount of the disinfectant liquid W is reserved in the bottom portion Pd of the reciprocating pipe 10 as to keep the air flow.

And, the air A is made contact with the disinfectant liquid W on the inner peripheral face Pe and in the bottom portion Pd to absorb foreign matter (matter to be removed) such as bacteria, pollen, dust, etc. included in the air A and to sterilize and clean the air A.

And, the disinfectant liquid W in the bottom portion

Pd is extracted and the flow piping P is washed with tap water for every predetermined period, and then, new disinfectant liquid W is dropped down.

Test results are shown in Table 8 below. The method for collecting and testing the air A is similar to that of the test example 1.

**[Table 8]**

| Test item | Air into the Suction opening | Air out of the Blow opening | Removal ratio |
|---|---|---|---|
| ①Bacteria, Fungi Number of | | | |
| B-coli | 185 | 3 | 98.4 % |
| Lactobacillus | 211 | 5 | 97.6 % |
| Fungi(spores) | 88 | 2 | 97.7 % |
| ②Number of Pollen/cm² (cedar, hinoki) | 195 | 2 | 99.0% |
| ③Carcasses of mites | 8 | 0 | 100% |
| ④degree of dust | (++++) | (-) | |
| ⑤Smell of | | | |
| Excrement | 3 | 1 | |
| Garbage | 3 | 0 ∼ 1 | |
| Ammonia | 3 | 1 | |
| Hydrogen sulfate | 3 | 1 | |
| Trimethylamine | 4 | 1 ∼ 2 | |

As clearly shown in Table 8 above, removal ratio of bacteria, fungi, pollen, etc. floating in the air A is more than 97%, and 100% of mites. Dust is also mostly removed, distinctive smell generated in daily life is slightly sensed or mostly deodorized. And, hair of companion animals can be also removed.

And, in Figure 3, the disinfectant liquid W is reserved in a tank T placed on the ground, and the disinfectant liquid W is sprayed or dropped from each of the upper portions of the going portion 11 and the returning portion 12 of the reciprocating pipe 10 through a pump 90 to keep the inner peripheral face Pe always wet. And, the physiology activating matter 39 is volatilized and mixed with the air A sent to the room R by the physiology activator 3. It can be said that this method is a method in which the sterilizer 2 in the embodiment shown in Figure 1 and Figure 2 is united with the reciprocating pipe 10.

The present invention can be modified. The places of installment of the fan F, the sterilizer 2, the physiology activator 3, the pump 90, the tank T, etc. can be freely set such as under the floor of buildings, basements, outside, machine rooms in building site, etc. And, the temperature control means 28 may be a heater and a cooling apparatus. And, in Figure 1 and Figure 3, although plural through holes are formed on the lower end portion of the partition wall of the going portion 11 and the returning portion 12 to keep air flow in the bottom portion Pd of the reciprocating pipe 10, the through holes may be replaced with notches. And, in the present invention, sterilization includes deactivation of viruses. The sterilizer 2 and the physiology activator 3 may be disposed on a conventional flow piping for air-conditioning with ground heat.

And, the present invention relates to the air-conditioning apparatus, having functionality (sterilization, air cleaning, dissolving stress, etc.) set (disposed) on the flow piping P, and method for the same. Especially, it shows power (high performance) when set (disposed) within a system utilizing ground heat. And, the sterilizer 2 and the physiology activator 3, not restricted to setting in the air-conditioning system utilizing ground heat, can be disposed on a normal air pipes (flow piping P) to obtain safe and beautifully clear air as shown in Tables 1 through 7.

As described above, clean, clear, and high quality germ and odor free air A, in which dust, pollen, carcasses of mite, hair of companion animals, etc. hardly exist, can be sent to the room R, and safe, pleasant, and healthy living space can be obtained because the functional air-conditioning apparatus of the present invention is a functional air-conditioning apparatus utilizing ground heat provided with the flow piping P which sucks the air A in the room R or outside S from the suction opening Pa, sends the air A into the ground G to make the air A heat-exchanged with the ground G, and blows the heat-exchanged air A into the room R through the blow opening Pb, in which the flow piping P is provided with the reciprocating pipe 10 for heat exchange having the going portion 11 to send the air A into the ground G and the returning portion 12 to return the air A heat-exchanged with ground heat under the ground; and the sterilizer 2, in which disinfectant liquid W is reserved, is disposed between the blow opening Pb and the ground exhaust opening 10b of the reciprocating pipe 10 for heat exchange. High level air cleanliness and healthy life can be obtained not only in houses, but also in offices and factories, and various large facilities. And, in comparison with conventional air-conditioning apparatus utilizing ground heat, further excellent functions are shown to contribute to popularization of air-conditioning apparatus utilizing ground heat.

And, the air A can be certainly made contact with the disinfectant liquid W, and foreign matter such as bacteria, dust, pollen, etc. can be certainly absorbed because the sterilizer 2 has the heat-insulating casing 21 for sterilization, the liquid reserve portion 22 in which the disinfectant liquid W is reserved, and the filter 24 of which one end side is dipped to the disinfectant liquid W in the liquid reserve portion 22 to suck the disinfectant liquid W by capillary phenomenon, and the sterilizer 2 has the intake guide portion 26 to induce the flowing air A to the liquid reserve portion 22 side between the inflow opening 21a of the casing 21 for sterilization and the filter 24. The air A can be efficiently and uniformly made contact with the disinfectant liquid W, and certain cleaning function can be stably obtained. Especially in summer, the air A is cooled by vaporization heat when passing the filter 24, and further energy saving effect (cooling effect) can be obtained.

And, the filter 24 is certainly kept wet and sterilization (deactivation of viruses) can be stably obtained because the filter 24 is composed of non-organic fiber of which thickness is set to be 0.01mm to 0.05mm, and the thickness of the filter 24 is set to be 0.05mm to 2.0mm. The air can be made contact with the disinfectant liquid W without reducing blowing efficiency (blowing ability). Capillary phenomenon can be certainly obtained without influence of blowing amount and wind power. The thickness, length, configuration, density of the fiber can be easily set in comparison with natural cotton and cellulose. The filter 24 deteriorates little, and durability is obtained.

And, high sterilization power can be obtained for a long period of time (excellent duration can be obtained) because the disinfectant liquid W has metal ion having antibacterial function, L-cysteine, and L-ascorbic acid as main components, and includes surface active agent except non-ion system. The disinfectant liquid W is excellent in permeation to bacteria, and rapid effect can be expected. The disinfectant liquid W is effective to allergic matters such as various bacteria and viruses, pollen, hair of companion animals, and excellent air cleaning effect can be obtained.

And, the air A can be obtained cooler in summer and warmer in winter in comparison with the air A heat-exchanged with the ground heat because the sterilizer 2 is provided with the temperature control means 28 to control the temperature of the disinfectant liquid W, and temperature of the air A blown out of the sterilizer 2 is controlled by the temperature control of the disinfectant liquid W.

And, effects of stress dissolving, recovery and promotion of natural healing power can be added to the sterilized and cleaned air A because the physiology activator 3, having the casing 31 for storage with heat insulation having the connection opening 31a in which the air A exhausted from the sterilizer 2, the storing portion 32 in which physiology activating matter 39 effective for resolving stress is stored, and an ultrasonic wave generating means 33 to volatilize the physiology activating matter 39 to be mixed with the air A, is disposed between the blow opening Pb and the sterilizer 2. Chronic unpleasant symptoms can be softened or dissolved, and recovery and promotion of natural healing power can be obtained.

And, clean, clear, and high quality germ and odor free air A, in which dust, pollen, carcasses of mite, hair of companion animals, etc. hardly exist, can be sent to the room R, and safe, pleasant, and healthy living space can be obtained because the functional air-conditioning method of the present invention is a functional air-conditioning method utilizing ground heat to send the air A in the room R or outside S into the ground G to make the air A heat-exchanged with the ground G, and send the heat-exchanged air A into the room R in which the air A is made contact with stored disinfectant liquid W to be sterilized and cleaned before being sent to the room R. High level air cleanliness and healthy life can be obtained not only in houses, but also in offices and factories, and various large facilities. And, in comparison with conventional air-conditioning method utilizing ground heat, further excellent functions are shown to contribute to popularization of air-conditioning apparatus utilizing ground heat.

And, the air A can be certainly made contact with the disinfectant liquid W, and foreign matter such as bacteria, dust, pollen, hair of companion animals, etc. can be certainly absorbed because the air A is induced to the disinfectant liquid W for contact on the ground side of a flow piping P in which the air A flows, and the air A is made pass through a filter 24 sucking the stored disinfectant liquid W by capillary phenomenon to be sterilized and cleaned. Especially in summer, the air A is cooled by vaporization heat when passing the filter 24, and further energy saving effect (cooling effect) can be obtained.

And, the air A can be made contact with the disinfectant liquid W uniformly for a long period of time to be certainly sterilized and cleaned because in the functional air-conditioning method utilizing ground heat to send the air A in the room R or outside S into the ground G to make the air A heat-exchanged with the ground G, and send the heat-exchanged air A into the room R, the inner peripheral face Pe of the flow piping P in which the air A flows is made wet with the disinfectant liquid W, and the air A is made contact with the disinfectant liquid W and flow in the flow piping P to be sterilized and cleaned. The method can easily correspond to a conventional flow piping P for heat exchange with the ground heat.

And, clean, clear, and high quality germ and odor free air A, in which dust, pollen, carcasses of mite, hair of companion animals, etc. hardly exist, can be sent to the room R, and safe, pleasant, and healthy living space can be obtained because the sterilizer 2, in which the disinfectant liquid W is stored, is disposed on the way of the flow piping P to exhaust the air A out of the blow opening Pb to the room R. High level air cleanliness and healthy life can be obtained not only in houses, but also in offices and factories, and various large facilities. And, in comparison with conventional air-conditioning apparatus, further excellent functions are shown.

### EXPLANATION OF THE MARKS

- 2: A sterilizer
- 3: A physiology activator
- 10: A reciprocating pipe for heat exchange
- 10b: A ground exhaust opening
- 11: A going portion
- 12: A returning portion
- 21: A casing for sterilization
- 21a: An inflow opening
- 22: A liquid reserve portion
- 24: A filter
- 26: An intake guide portion
- 28: A temperature control means
- 31: A casing for storage
- 31a: A connection opening
- 32: A storing portion
- 33: An ultrasonic wave generating means
- 39: Physiology activating matter
- A: Air
- G: Ground
- P: A flow piping
- Pa: A suction opening
- Pb: A blow opening
- Pe: An inner peripheral face
- R: A room
- S: Outside
- W: Disinfectant liquid

## Claims

1. A functional air-conditioning apparatus utilizing ground heat provided with a flow piping (P) which sucks air (A) in a room (R) or outside (S) from a suction opening (Pa), sends the air (A) into ground (G) to make the air (A) heat-exchanged with the ground (G), and blows the heat-exchanged air (A) into the room (R) through a blow opening (Pb) **characterized by** that:
the flow piping (P) is provided with a reciprocating pipe (10) for heat exchange having a going portion (11) to send the air (A) into the ground (G) and a returning portion (12) to return the air (A) heat-exchanged with ground heat under the ground; and
a sterilizer (2), in which disinfectant liquid (W) is reserved, is disposed between the blow opening (Pb) and a ground exhaust opening (10b) of the reciprocating pipe (10) for heat exchange.

2. The functional air-conditioning apparatus as set forth in claim 1, wherein the sterilizer (2) has a heat-insulating casing (21) for sterilization, a liquid reserve portion (22) in which the disinfectant liquid (W) is reserved, and a filter (24) of which one end side is dipped to the disinfectant liquid (W) in the liquid reserve portion (22) to suck the disinfectant liquid (W) by capillary phenomenon; and
the sterilizer (2) has an intake guide portion (26) to induce the flowing air (A) to the liquid reserve portion (22) side between an inflow opening (21a) of the casing (21) for sterilization and the filter (24).

3. The functional air-conditioning apparatus as set forth in claim 2, wherein the filter (24) is composed of non-organic fiber of which thickness is set to be 0.01mm to 0.05mm, and the thickness of the filter (24) is set to be 0.05mm to 2.0mm.

4. The functional air-conditioning apparatus as set forth in claim 1, 2, or 3, wherein the disinfectant liquid (W) has metal ion having antibacterial function, L-cysteine, and L-ascorbic acid as main components, and includes surface active agent except non-ion system.

5. The functional air-conditioning apparatus as set forth in claim 1, 2, 3, or 4, wherein the sterilizer (2) is provided with a temperature control means (28) to control the temperature of the disinfectant liquid (W); and
temperature of the air (A) blown out of the sterilizer (2) is controlled by the temperature control of the disinfectant liquid (W).

6. The functional air-conditioning apparatus as set forth in claim 1, 2, 3, 4, or 5, wherein a physiology activator (3), having a casing (31) for storage with heat insulation having a connection opening (31a) in which the air (A) exhausted from the sterilizer (2), a storing portion (32) in which physiology activating matter (39) effective for resolving stress is stored, and an ultrasonic wave generating means (33) to volatilize the physiology activating matter (39) to be mixed with the air (A), is disposed between the blow opening (Pb) and the sterilizer (2).

7. A functional air-conditioning method utilizing ground heat to send air (A) in a room (R) or outside (S) into ground (G) to make the air (A) heat-exchanged with the ground (G), and send the heat-exchanged air (A) into the room (R) **characterized by** that:
the air (A) is made contact with stored disinfectant liquid (W) to be sterilized and cleaned before being sent to the room (R).

8. The functional air-conditioning method as set forth in claim 7, wherein the air (A) is induced to the disinfectant liquid (W) for contact on the ground side of a flow piping (P) in which the air (A) flows, and the air (A) is made pass through a filter (24) sucking the stored disinfectant liquid (W) by capillary phenomenon to be sterilized and cleaned.

9. A functional air-conditioning method utilizing ground heat to send air (A) in a room (R) or outside (S) into ground (G) to make the air (A) heat-exchanged with the ground (G), and send the heat-exchanged air (A) into the room (R) **characterized by** that:
an inner peripheral face (Pe) of the flow piping (P) in which the air (A) flows is made wet with the disinfectant liquid (W); and
the air (A) is made contact with the disinfectant liquid (W) and flow in the flow piping (P) to be sterilized and cleaned.

10. A functional air-conditioning apparatus **characterized by** that a sterilizer (2), in which disinfectant liquid (W) is stored, is disposed on the way of a flow piping (P) to exhaust air (A) out of a blow opening (Pb) to a room (R).
